(19) Europäisches Patentamt
European Patent Office
Office européen des brevets

(11) Veröffentlichungsnummer : **0 068 261**
**B1**

(12) **EUROPÄISCHE PATENTSCHRIFT**

(45) Veröffentlichungstag der Patentschrift : **03.04.85**

(51) Int. Cl.⁴ : **C 07 D405/14**, A 61 K 31/445

(21) Anmeldenummer : **82105174.5**

(22) Anmeldetag : **14.06.82**

(54) **N-Oxacyclyl-Alkylpiperidin-Derivate, Verfahren zu ihrer Herstellung, sie enthaltende pharmazeutische Präparate und deren Verwendung.**

(30) Priorität : **20.06.81 DE 3124366**

(43) Veröffentlichungstag der Anmeldung : **05.01.83 Patentblatt 83/01**

(45) Bekanntmachung des Hinweises auf die Patenterteilung : **03.04.85 Patentblatt 85/14**

(84) Benannte Vertragsstaaten : **AT BE CH DE FR GB IT LI LU NL SE**

(56) Entgegenhaltungen :
**EP-A- 0 004 358**
**EP-A- 0 005 610**
**FR-A- 2 213 059**
**FR-A- 2 361 889**
**CHEMICAL ABSTRACTS, Band 84, Nr. 5, 2. Dezember 1976, Seite 466, Nr. 31060h, Columbus, Ohio, USA**
**CHEMICAL ABSTRACTS, Band 84, Nr. 5, 2, Dezember 1976, Seite 466, Nr. 31059q, Columbus, Ohio, USA**
**CHEMICAL ABSTRACTS, Band 91, Nr. 21, 19. November 1979, Seiten 33,34, Nr. 168329s, Columbus, Ohio, USA**

(73) Patentinhaber : **HOECHST AKTIENGESELLSCHAFT**
**Postfach 80 03 20**
**D-6230 Frankfurt am Main 80 (DE)**

(72) Erfinder : **Henning, Rainer, Dr.**
**Völklinger Weg 56**
**D-6000 Frankfurt am Main 71 (DE)**
Erfinder : **Lattrell, Rudolf, Dr.**
**Heuhohlweg 6H**
**D-6240 Königstein/Taunus (DE)**
Erfinder : **Gerhards, Hermann, Dr.**
**Wacholderweg 4**
**D-6238 Hofheim am Taunus (DE)**

EP 0 068 261 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Nach der Europäischen Patentanmeldung 0 004 358 sind N-Oxacylyl-alkylpiperidine mit stimulierenden und antidepressiven Wirkungen bekannt.

Die neuen Verbindungen, die den Gegenstand der vorliegenden Erfindung bilden, unterscheiden sich von den bekannten Verbindungen bezüglich ihrer Struktur durch die Methylengruppe zwischen den beiden Ringsystemen, durch den an den Imidazolidinonring ankondensierten Phenylring bzw. durch dessen Substitution. Bezüglich ihrer Wirkungen unterscheiden sie sich ebenfalls ; die neuen Verbindungen wirken antipsychotisch und neuroleptisch.

Gegenstand der Erfindung sind neue Ve·bindungen der Formel I

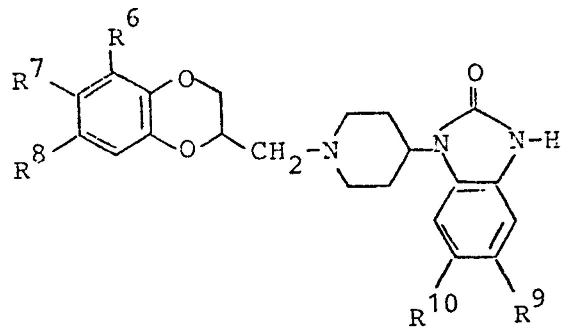

(I)

in der $R^6$ Wasserstoff oder Methyl, $R^7$ und $R^8$ unabhängig voneinander Wasserstoff, Fluor, Chlor oder Methyl, $R^9$ Fluor, Chlor, Methyl oder Methoxy und $R^{10}$ Wasserstoff oder Methyl bedeuten, vor allem solche Verbindungen, in denen $R^6$, $R^7$ und $R^{10}$ Wasserstoff, $R^8$ Wasserstoff oder Fluor und $R^9$ Fluor, Chlor, Methyl oder Methoxy bedeuten.

Gegenstand der Erfindung ist weiter ein Verfahren zur Herstellung der Verbindungen gemäß Formel (I), das dadurch gekennzeichnet ist, daß man

(1) einen reaktionsfähigen Ester eines oxacyclischen Alkanols der allgemeinen Formel (II) mit einem Piperidin-Derivat der allgemeinen Formel (III) kondensiert.

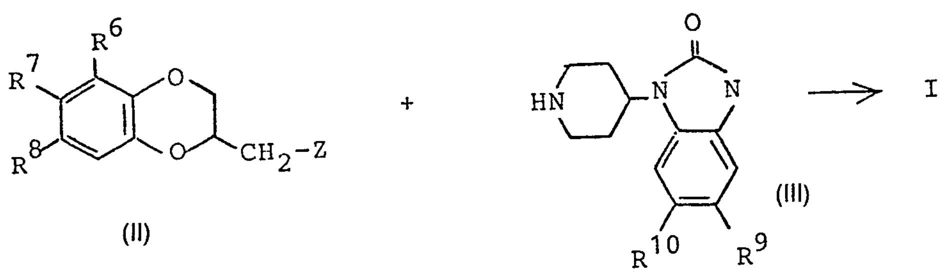

(II) (III)

$R^6$, $R^7$ und $R^8$ in Formel (II) und $R^9$ und $R^{19}$ in Formel (III) haben die gleichen Bedeutungen wie in Formel (I). Der Rest Z in Formel (II) steht dabei für Halogen wie Chlor, Brom oder Jod, oder für eine reaktionsfähige Estergruppe wie den Schwefelsäurerest oder die p-Toluolsulfonyl-, m-Brombenzolsulfonyl-, p-Nitrobenzolsulfonyl-, Methansulfonyl- oder Trifluormethansulfonylgruppe.

Ein weiteres Verfahren ist dadurch gekennzeichnet, daß man

(2) eine Verbindung der Formel (IV)

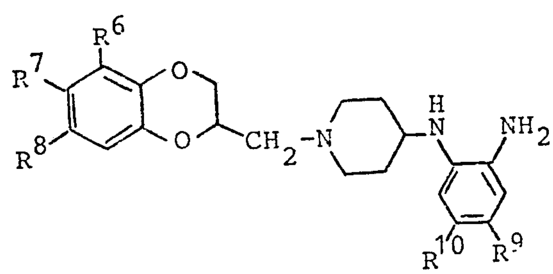

(IV)

mit einem reaktiven Kohlensäurederivat umsetzt, wobei alle Substituenten die vorstehend genannten Bedeutungen haben.

Unter reaktiven Kohlensäurederivaten werden in der vorliegenden Erfindung Ammoniumcyanat oder -thiocyanat, ein Metallcyanat oder -thiocyanat, ein Halogencyan, ein Cyanamid, gegebenenfalls durch Alkyl mit 1 bis 4 Kohlenstoffatomen substituiert, Schwefelkohlenstoff oder Carbonylsulfid, ein Kohlensäurehalogenid, ein Thiokohlensäurehalogenid, ein Chlorkohlensäurealkyl-, -benzyl- oder -phenylester, ein Kohlensäuredialkyl-, -dibenzyl- oder -diphenylester, Harnstoff, Thioharnstoff, 1,1-Carbonyldiimidazol, ein O-Alkylisoharnstoff, ein S-Alkylisothioharnstoff sowie Guanidin verstanden.

Die Kondensation nach Verfahren (1) wird in einem organischen Lösungsmittel wie Methanol, Ethanol, Toluol, Dioxan, Tetrahydrofuran, Pyridin, Dimethylsulfoxid, Dimethylformamid, vorzugsweise in Gegenwart eines basischen Kondensationsmittels, durchgeführt. Als solche Kondensationsmittel kommen Alkali- und Erdalkalihydroxide, -carbonate sowie -hydrogencarbonate in Betracht, außerdem Alkalimetallhydride, -alkoxide und -alkanoate sowie organische tertiäre Stickstoffbasen wie Triethylamin, Tripropylamin, N-Methylmorpholin oder Lutidin. Die Reaktionstemperatur beträgt üblicherweise 0 °C bis 180 °C, vorzugsweise 20° bis 100 °C.

Verfahren (2) wird in an sich bekannter Weise ohne Lösungsmittel oder in einem organischen Lösungsmittel oder einer Mischung, bestehend aus einem organischen Lösungsmittel mit Wasser durchgeführt. Handelt es sich bei dem reaktiven Kohlensäurederivat um ein Ammonium- oder Metallthiocyanat bzw. -cyanat, so kommen als Lösungsmittel solche in Betracht, die polar und mit Wasser mischbar sind, wie Methanol, Ethanol, Aceton, Tetrahydrofuran, Dioxan, Dimethylformamid, Dimethylsulfoxid sowie deren Mischungen mit Wasser.

In allen anderen Fällen kommen die eben genannten wasserfreien Lösungsmittel sowie zusätzlich Toluol, Xylol, Glykoldimethylether, Diglykoldimethylether, Triglykoldimethylether, Pyridin sowie Sulfolan in Betracht. Zusätzlich kann ein basisches Kondensationsmittel zum Entzug der freiwerdenden Säure zugesetzt werden. Als solche kommen vorzugsweise die bei Verfahren (1) genannten in Betracht.

Die Ausgangsstoffe für die beschriebenen Verfahren werden nach literaturbekannten Methoden hergestellt. Die Verbindungen der Formel (II) erhält man aus einem substituierten Brenzcatechin durch Umsetzung mit Epichlorhydrin und anschließende Umwandlung des so erhaltenen Oxacyclyl-alkanols in den reaktiven Ester mit Hilfe einer starken Säuren oder deren Derivate wie Thionylchlorid, einem Phosphorhalogenid oder einem Benzolsulfonylhalogenid (J. Med. Chem. *8*, 446 (1965)).

Verbindungen der Formel (III) können z. B. nach der deutschen Offenlegungsschrift 2 526 393 oder der deutschen Offenlegungsschrift 2 400 094, ausgehend von in 1-Stellung mit einer Carbethoxy- oder Benzylgruppe geschütztem Aminopiperidin und einem geeignet substituierten o-Chlornitrobenzol, anschließende katalytische Hydrierung der Nitrogruppe zur Aminogruppe, z. B. unter Verwendung von Raney-Nickel als Katalysator, Cyclisierung mit einem Kohlensäurederivat analog Verfahren (2) und Abspaltung der Aminoschutzgruppe in 1-Stellung, hergestellt werden.

Verbindungen der Formel (IV) erhält man durch Umsetzung von Verbindungen der Formel (II) mit Verbindungen der Formel (VII),

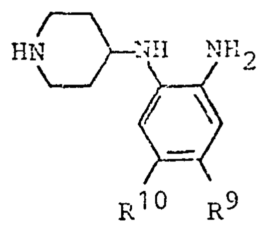

(VII)

die bei der Herstellung der Ausgangsstoffe der Formel (III) als Zwischenprodukte anfallen. Die Reaktionsbedingungen für diese Reaktion sind die gleichen wie bei Verfahren (1) beschrieben. Im folgenden beziehen sich Prozentangaben auf das Gewicht, wenn nichts anderes angegeben ist.

Die erfindungsgemäßen Verbindungen der Formel (I) zeigen pharmakologische Wirkungen, insbesondere antipsychotische (neuroleptische) Wirkungen. So antagonisieren sie dosisabhängig die Amphetamin-Aggregations-Toxizität bei Mäusen. In diesem Test erhalten Gruppen von je 10 Mäusen, die auf engem Raum zusammensitzen (ca. 25 $cm^2$/Maus), eine Stunde nach Gabe einer Verbindung der Formel (I) 20 mg/kg D-Aphetamin in 0,2 %iger wäßriger Lösung subcutan injiziert. Es wird dabei diejenige Dosis der Verbindung (I) ermittelt, die 50 % der Tiere vor dem Tod durch die Amphetamin-Vergiftung schützt. Die $ED_{50}$-Werte der Verbindungen der Formel (I) liegen zwischen 0,01 und 40 mg/kg. Weiterhin inhibieren sie das durch Amphetamin und Apomorphin bei Ratten hervorgerufene stereotypische Verhalten. Die Wirkung wird durch übliche statistische Methoden bestimmt ; mit diesen werden die $ED_{50}$-Werte für die Tests errechnet. Sie liegen für die Verbindungen der Formel (I) bei 0,1 bis 20 mg/kg, vorzugsweise bis 5 mg/kg, für die Inhibierung der Amphetamin- und bei 0,01 bis 20 mg/kg, vorzugsweise bis 5 mg/kg, für die Inhibierung der Apomorphin-induzierten Stereotypien.

In einem weiteren Versuch wird bei Ratten eine einseitige Hirnläsion erzeugt, worauf sich die Tiere nach Gabe von Amphetamin in der Richtung der Läsion um ihre Achse drehen. Die Verbindungen der Formel (I) inhibieren dieses Verhalten mit $ED_{50}$-Werten von 0,05 bis 20 mg/kg, vorzugsweise bis 5 mg/kg.

Weiterhin inhibieren die Verbindungen der Formel (I) stark die intracranielle Selbststimulation von Ratten ($ED_{50}$-Werte von 0,01 bis 10 mg/kg, vorzugsweise bis 5 mg/kg).

Bei diesem Verfahren können sich Ratten, denen Elektroden ins Gehirn implantiert wurden, auf Tastendruck selbst Reize setzen. Bestimmt wird die Dosis der Verbindungen, die die Zahl der Tastendrücke um 50 % reduziert.

Die Verbindungen haben keine oder nur eine schwache kataleptogene Wirkung, d. h. sie bewirken nur in hohen Dosen (> 30 mg/kg) eine kataleptische Starre bei Ratten. Daneben bewirken sie bis zu hohen Dosierungen (> 10 mg/kg intraperitoneal bei der Maus) keine oder nur eine geringe Blockierung von α- sowohl als auch β-adrenergen Rezeptoren, sind also nicht sympatholytisch wirksam.

Die oben angegebenen pharmakologischen Wirkungen sind denen bekannter Neuroleptika deutlich überlegen. So liegt zum Beispiel das Verhältnis von wirksamer Dosis zu kataleptogener Dosis bei 1 : 20 bis 1 : 80 je nach Testverfahren. Für die gebräuchlichen Butyrophenon-Derivate wie Haloperidol und Phenothiazine wie Chlorpromazin liegt dieses Verhältnis bei 1 : 4 bis 1 : 8 (P.A.J. Janssen u. W.F.M. van Bever in G. Stille, H. Hoffmeister (Herausgeber) Psychotropic Agents, Vol. I, Springer 1980). Die kataleptogenen Wirkungen führen in der Anwendung zu nicht erwünschten extrapyramidalen Störungen. Daneben weisen die bekannten Neuroleptika häufig eine α-sympatholytische Nebenwirkung auf, die unter anderem zu einer unerwünschten Blutdrucksenkung führt.

Die erfindungsgemäßen Verbindungen und ihre pharmakologisch verträglichen Salze sind innerhalb eines breiten Dosierungsbereiches wirksam. Die Höhe der verabreichten Dosierung ist abhängig von der Art der gewünschten Behandlung, von der Verabreichungsweise, vom Zustand, vom Typ und von der Größe des zu behandelnden Säugetieres. Bei oraler Dosierung werden befriedigende Ergebnisse mit Dosen von 0,01 bis 100 mg, vorzugsweise bis 10 mg, einer Verbindung der Formel I pro kg Tierkörpergewicht erzielt. Beim Menschen variiert die tägliche Dosis zwischen 10 mg und 800 mg, vorzugsweise 20 bis 500 mg, wobei Einzeldosen von 5 bis 200 mg vorzugsweise ein- bis dreimal täglich gegeben werden können. Für intravenöse und intramuskuläre Anwendung beträgt die Dosis 1 bis 300 mg, vorzugsweise 5 bis 150 mg täglich.

Die pharmakologisch verwendbaren Verbindungen der vorliegenden Erfindung und ihre Salze können zur Herstellung von pharmazeutischen Präparaten verwendet werden, welche eine wirksame Menge der Aktivsubstanz zusammen mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, zum Beispiel Lactose, Dextrose, Rohrzucker, Mannitol, Sorbitol, Cellulose und/oder Glycin und Schmiermitteln, zum Beispiel Kieselerde, Talk, Stearinsäure oder deren Salze, wie Mangesium- oder Calciumstearat, und/oder Polyethylenglykol enthalten. Tabletten enthalten ebenfalls Bindemittel, zum Beispiel Magnesiumaluminiumsilicat, Stärke-Paste, Gelatine, Traganth, Methylcellulose, Natriumcarboxymethylcellulose und/oder Polyvinylpyrrolidon und, falls benötigt, Farbstoffe, Geschmacksstoffe oder Süßmittel. Injizierbare Lösungen sind vorzugsweise isotonische wäßrige Lösungen oder Suspensionen, die sterilisert sein können und Hilfsstoffe wie Konservier-, Stabilisierungs-, Netz- und/oder Emulgiermittel, Löslichkeitsvermittler, Salze zur Regulierung des osmotischen Drucks und/oder Puffersubstanzen enthalten können. Die erfindungsgemäßen pharmazeutischen Präparate, die, wenn erwünscht, weitere pharmakologisch wertvolle Stoffe enthalten können, werden in an sich bekannter Weise, zum Beispiel mittels konventioneller Misch-, Granulier- oder Dragierverfahren, hergestellt und enthalten etwa 0,1 % bis etwa 75 %, bevorzugt etwa 1 % bis etwa 50 % des Wirkstoffs.

Die im Anschluß folgenden Beispiele sollen die Erfindung erläutern : Prozentangaben beziehen sich auf das Gewicht, wenn nichts anderes angegeben ist.

## Beispiel 1

1-[(Benzo-1,4-dioxan-2-yl)-methyl]-4-(5-chlor-benzimidazol-2-on-1-yl)-piperidin-Hydrochlorid

1.1 1-Carbethoxy-4-(2-nitro-4-chlor-anilino)-piperidin

Ein Gemisch aus 0,25 Mol 4-Amino-1-piperidincarbonsäureethylester, 0,3 Mol 1,4-Dichlor-2-nitrobenzol, 0,3 Mol Natriumcarbonat, 0,2 g Kaliumjodid und 160 ml Cyclohexanol wird 40 Stunden bei 150 °C gerührt. Nach Abkühlen wird mit Toluol und Wasser versetzt, die organische Schicht abgetrennt, dreimal mit Wasser gewaschen, getrocknet und eingedampft. Der ölige Rückstand wird in heißem Diisopropylether gelöst, die Lösung wird mit Aktivkohle unter Rückfluß gerührt, filtriert und kristallisieren gelassen, F., 116 °C.

1.2 1-Carboethoxy-4-(2-amino-4-chlor-anilino)-piperidin

Eine Lösung von 59 g 1-Carbethoxy-4-(2-nitro-4-chlor-anilino)-piperidin in 270 ml THF und 96 ml absolutem Ethanol wird bei Normaldruck und Raumtemperatur mit 15 g Raney-Nickel als Katalysator hydriert. Nach Beendigung der Wasserstoffaufnahme wird filtriert und eingedampft, F. 150 °C.

1.3 1-Carbethoxy-4-(5-chlor-benzimidazol-2-on-1-yl)-piperidin

Eine Mischung aus 24,7 g 1-Carbethoxy-4-(2-amino-4-chlor-anilino)-piperidin und 7,2 g Harnstoff wird 3,5 Stunden auf 160-180 °C erhitzt. Die Schmelze wird in 250 ml Toluol aufgenommen und so lange unter Rühren erhitzt, bis alles in Lösung gegangen ist. Die Lösung wird mit Aktivkohle geklärt, filtriert und auf 50 ml eingeengt. Das Produkt wird mit Diisopropylether ausgefällt, F. 160 °C.

1.4 4-(5-Chlor-benzimidazol-2-on-1-yl)-piperidin

Eine Mischung aus 22,3 g 1-Carbethoxy-4-(5-chlor-benzimidazol-2-on-1-yl)-piperidin, 13 g 50 %iger Natronlauge und 90 ml Wasser wird 24 Stunden am Rückfluß gekocht. Nach Abkühlen wird die Lösung 30 Minuten mit 8,5 g Ammoniumchlorid gerührt, mit Chloroform extrahiert, das Ungelöste abfiltriert und verworfen. Die Chloroformlösung wird getrocknet und eingedampft. Das Produkt kristallisiert und schmilzt bei 220 °C.

Nach den Vorschriften von Beispiel 1.1 bis 1.4 werden analog die folgenden Ausgangsstoffe aus den entsprechend substituierten o-Chlor-nitrobenzolen hergestellt :

4-(5-Methyl-benzimidazol-2-on-1-yl)-piperidin, F. 196-197 °C
4-(5-Methoxy-benzimidazol-2-on-1-yl)-piperidin, F. 117 °C (Zers.)
4-(5,6-Dimethyl-benzimidazol-2-on-1-yl)-piperidin
4-(6-Methyl-benzimidazol-2-on-1-yl)-piperidin
4-(5-Fluor-benzimidazol-2-on-1-yl)-piperidin, F. 159 °C.

1.5 2-Hydroxymethyl-1,4-benzodioxan

0,5 Mol Brenzcatechin werden bei 100 °C heftig mit 1,5 Mol Epichlorhydrin und 1 Mol 10 %iger wäßrige Kalilauge gerührt. Nach Abkühlen wird mit Ether extrahiert, der Etherextrakt wird mit verdünnter Kalilauge und Wasser gewaschen und getrocknet, das Produkt wird nach Eindampfen aus Ethanol umkristallisiert, F. 87-90 °C.

1.6 2-(p-Toluolsulfonyloxymethyl)-1,4-benzodioxan

0,35 Mol 2-Hydroxymethyl-1,4-benzodioxan werden in 200 ml wasserfreiem Pyridin gelöst und mit 0,38 Mol p-Toluolsulfonylchlorid versetzt. Nach 16 Stunden bei Raumtemperatur wird mit eiskalter verdünnter Salzsäure angesäuert und mit Ether extrahiert. Nach Trocknen und Einengen wird umkristallisiert, F. 79 °C.

Analog den vorstehenden Vorschriften wurden die folgenden Verbindungen hergestellt :

2-(p-Toluolsulfonyloxymethyl)-5-methyl-1,4-benzodioxan, F. 80-81 °C
2-(p-Toluolsulfonyloxymethyl)-6-fluor-1,4-benzodioxan, Öl
2-(p-Toluolsulfonyloxymethyl)-7-fluor-1,4-benzodioxan, F. 87-89 °C
2-(p-Toluolsulfonyloxymethyl)-6,7-dimethyl-1,4-benzodioxan, F. 93,5-95 °C

1.7 2-Chlormethyl-1,4-benzodioxan

0,42 Mol 2-Hydroxymethyl-1,4-benzodioxan werden mit 0,42 Mol Thionylchlorid in 600 ml trockenem Pyridin 3 h auf 100 °C erhitzt. Nach Abkühlen wird mit eiskalter verdünnter Salzsäure versetzt und mit Ether extrahiert, Das Lösungsmittel wird nach Trocknen entfernt, der Rückstand im Vakuum destilliert, Sdp. 80 °C (0,9 mbar).

Nach dieser Vorschrift wurden ebenfalls die folgenden Verbindungen synthetisiert :

2-Chlormethyl-6-methyl-1,4-benzodioxan, Sdp. 86-100 °C (0,2 mbar)
2-Chlormethyl-7-methyl-1,4-benzodioxan, Sdp. 96-102 °C (0,5 mbar)
2-Chlormethyl-7-chlor-1,4-benzodioxan, Sdp. 95-96 °C (0,4 mbar)

1.8 2-Brommethyl-1,4-benzodioxan

0,3 Mol 2-Hydroxymethyl-1,4-benzodioxan wird langsam mit 0,11 Mol Phosphortribromid in 500 ml Tetrachlorethan versetzt und 2 Stunden auf 80-90 °C erwärmt. Nach Abkühlen wird in Wasser gegossen, die organische Schicht abgetrennt, mit verdünnter Natronlauge und Wasser gewaschen, getrocknet, eingeengt und destilliert, Sdp. 102-103 °C (1,3 mbar).

Nach dieser Vorschrift wurde weiterhin hergestellt :

2-Brommethyl-6,7-dichlor-1,4-benzodioxan, Sdp. 124-127 °C (0,3 mbar)

1.9 1-[(Benzo-1,4-dioxan-2-yl)-methyl]-4-(5-chlor-benzimidazol-2-on-1-yl)-piperidin · Hydrochlorid

2,3 g 2-Brommethyl-1,4-benzodioxan, 2,2 g 4-(5-Chlor-benzimidazol-2-on-1-yl)-piperidin, 1,4 ml Triethylamin und 0,3 g Natriumjodid werden in 25 ml trockenem Dimethylformamid 60 Stunden bei Raumtemperatur gerührt, dann auf Wasser gegossen ; Diisopropylether wird zugegeben und die Mischung 2 Stunden heftig gerührt. Das ausgefallene Produkt wird abgesaugt, mit Wasser und Diisopropylether gewaschen und getrocknet ; die so erhaltene freie Base schmilzt bei 226-228 °C.

NMR : 11,0 s (1H) ; 7,3-6,6 m (7H) ; 4,5-3,6 m (4H) ; 3,3-1,5 m (10H)

IR : 1 685 $cm^{-1}$

Hydrochlorid : Die Base wird in Methylenchlorid/Methanol gelöst. 3 ml 2,5-normaler ethanolischer HCl werden zugegeben, zur Trockene eingeengt, zweimal in Aceton aufgenommen und jeweils zur Trockene eingeengt ; farbloses Pulver vom F. 196-200 °C (Zersetzung).

Analyse Ber. C 57,8 H 5,3
Gef. C 57,7 H 5,7

Analog der in Beispiel 1.9 angegebenen Vorschrift werden unter Verwendung entsprechender Mengen der betreffenden Ausgangsmaterialien die folgenden Verbindungen hergestellt :

Beispiel 2

1-[(5-Methyl-benzo-1,4-dioxan-2-yl)-methyl]-4-(5-chlorbenzimidazol-2-on-1-yl)-piperidin

NMR : 10,5 s (1H) ; 7,3-6,8 m (6H) ; 4,6-4,0 m (4H) ; 3,3-1,5 m + s (13H).
IR : 1 685 $cm^{-1}$

Beispiel 3

1-[(6-Methyl-benzo-1,4-dioxan-2-yl)-methyl]-4-(5-chlorbenzimidazol-2-on-1-yl)-piperidin

Freie Base : F. 164-166 °C
NMR : 10,8 S (1H) ; 7,4-6,6 m (6H) ; 4,6-3,9 m (4H) ; 3,3-1,5 m + s (13H)
IR : 1 691 $cm^{-1}$
Hydrochlorid : F. 224-226 °C (Zers.)

Beispiel 4

1-[(7-Methyl-benzo-1,4-dioxan-2-yl)-methyl]-4-(5-chlorbenzimidazol-2-on-1-yl)-piperidin

NMR : 10,6 s (1H)) ; 7,4-6,6 m (6H) ; 4,5-3,9 m (4H) ; 3,3-1,5 m + s (13H)
IR : 1 685 $cm^{-1}$

Beispiel 5

1-[(6,7-Dimethyl-benzo-1,4-dioxan-2-yl)-methyl]-4-(5-chlorbenzimidazol-2-on-1-yl)-piperidin

NMR : 10,8 S (1H) ; 7,4-6,6 M (5H) ; 4,5-3,9 M (4H) ; 3,3-1,4 m + 2 s (16H)
IR : 1 683 $cm^{-1}$

Beispiel 6

1-[(7-Chlor-benzo-1,4-dioxan-2-yl)-methyl]-4-(5-chlorbenzimidazol-2-on-1-yl)-piperidin

Freie Base : F. 184-189 °C
NMR : 11,0 s (1H) ; 7,4-6,6 m (6H) ; 4,5-3,9 m (4H) ; 3,5-1,5 M (10H)
IR : 1 683 $cm^{-1}$
Hydrochlorid : F. 180 °C (Zers.)

Beispiel 7

1)[(7-Chlor-benzo-1,4-dioxan-2-yl)-methyl]-4-(5-methylbenzimidazol-2-on-1-yl)piperidin

Freie Base : F. 187 °C
NMR : 10,9 S (1H) ; 7,4-6,7 m (6H) ; 4,5-3,9 m (4H) ; 3,5-1,5 m + s (13H)
IR : 1 686 $cm^{-1}$
Hydrochlorid : F. 245 °C (Zers.)

Beispiel 8

1-[(6,7-Dichlor-benzo-1,4-dioxan-2-yl)-methyl]-4-(5-chlorbenzimidazol-2-on-1-yl)piperidin

Freie Base : F. 238-240 °C
NMR : 10,7 s (1H) ; 7,4-6,7 m (5H) ; 4,5-3,9 m (4H) ; 3,5-1,5 m (10H)
IR : 1 682 $cm^{-1}$
Hydrochlorid : F. 289-290 °C

Beispiel 9

1-[(6,7-Dichlor-benzo-1,4-dioxan-2-yl)-methyl)]-4-(5-methylbenzimidazol-2-on-1-yl)-piperidin

NMR : 11,1 s (1H) ; 7,4-6,7 m (5H) ; 4,5-3,9 m (4H) ; 3,6-1,4 m + s (13H)
IR : 1 690 $cm^{-1}$

Beispiel 10

1-[(Benzo-1,4-dioxan-2-yl)-methyl]-4-(5-methyl-benzimidazol-2-on-1-yl)-piperidin

10.1 4-(4-Methyl-2-nitro-anilino)-piperidin-Hydrobromid

30 g 1-Carbethoxy-4-(4-methyl-2-nitro-anilino)-piperidin in 400 ml einer 48 %igen Bromwasserstoffsäure werden 4 Stunden unter Rühren am Rückfluß gekocht. Nach Abkühlen wird der gebildete Niederschlag abgesaugt und mit Wasser und Petrolether gewaschen. Man erhält das Produkt als hellbeiges Pulver.

10.2 1-[(Benzo-1,4-dioxan-2-yl)-methyl]-4-(4-methyl-2-nitroanilino)-piperidin

11,5 g 2-Brommethyl-1,4-benzodioxan, 20,4 g 4-(4-Methyl-2-nitro-anilino)-piperidin-Hydrobromid, 20 g Kaliumcarbonat und 0,5 g Kaliumjodid werden in 120 ml Methylisopropylketon gelöst und 24 Stunden am Rückfluß erhitzt. Nach Abkühlen wird mit Wasser versetzt, die organische Phase abgetrennt, getrocknet und eingeengt.

10.3 1-[(Benzo-1,4-dioxan-2-yl)-methyl]-4-(4-methyl-2-amino-anilino)-piperidin

20 g 1-[(Benzo-1,4-dioxan-2-yl)-methyl]-4-(4-methyl-2-nitro-anilino)-piperidin werden unter Verwendung von 4 g Raney-Nickel als Katalysator in 200 ml Ethanol bei Normaldruck und Raumtemperatur hydriert. Nach Beendigung der Wasserstoffaufnahme wird filtriert, mit Ethanol nachgewaschen und eingeengt.

10.4 1-[(Benzo-1,4-dioxan-2-yl)-methyl]-4-(5-methyl-benzimidazol-2-on-1-yl)-piperidin

14,5 g 1-[(Benzo-1,4-dioxan-2-yl)-methyl]-4-(4-methyl-2-amino-anilino)-piperidin in 5 ml 10 %iger Salzsäure und 40 ml Wasser werden unter Kühlen und Rühren mit einer Lösung von 4,8 g Kaliumcyanat in 10 ml Wasser versetzt.

Nach Beenden der Zugabe wird 1 Stunde bei Raumtemperatur und anschließend 24 Stunden bei Rückflußtemperatur gerührt. Nach Abkühlen wird mit Chloroform extrahiert. Der Extrakt wird mit 5 %iger Salzsäure gewaschen, getrocknet, filtriert und eingeengt, F. 209-215 °C.

NMR : 10,3 s (1H) ; 7,4-6,8 m (7H) ; 4,6-4,0 m (4H) ; 3,3-1,4 m + s (13H)
IR : 1 691 $cm^{-1}$
Hydrochlorid : F. 212-214 °C

Auf gleiche Weise werden aus den entsprechenden Ausgangsstoffen die folgenden Verbindungen hergestellt :

Beispiel 11

1-[(Benzo-1,4-dioxan-2-yl)-methyl]-4-(5,6-dimethyl-benzimidazol-2-on-1-yl)-piperidin

NMR : 10,4 s (1H) ; 7,1-6,5 m (6H) ; 4,6-4,0 m (4H) ; 3,5-1,5 m + s (16H)
IR : 1 678 $cm^{-1}$

Beispiel 12

1-[(Benzo-1,4-dioxan-2-yl)-methyl]-4-(6-methyl-benzimidazol-2-on-1-yl)-piperidin

NMR : 10,8 s (1H) ; 7,2-6,6 m (7H) ; 4,7-4,0 m (4H) ; 3,4-1,3 m + s (13H)
IR : 1 682 $cm^{-1}$

Beispiel 13

1-[(5-Methyl-benzo-1,4-dioxan-2-yl)-methyl]-4-(5-methyl-benzimidazol-2-on-1-yl)-piperidin

NMR : 10,9 s (1H) ; 7,2-6,5 m (6H) ; 4,7-4,0 m (4H) ; 3,4-1,3 m + s (16H)

Beispiel 14

1-[(6-Methyl-benzo-1,4-dioxan-2-yl)-methyl]-4-(6-methyl-benzimidazol-2-on-1-yl)-piperidin

NMR : 10,8 s (1H) ; 7,2-6,5 m (6H) ; 4,6-4,0 m (4H) ; 3,5-1,4 m + 2 s (16H)
IR : 1 688 $cm^{-1}$

Beispiel 15

1-[(6,7-Dimethyl-benzo-1,4-dioxan-2-yl)-methyl]-4-(5-methyl-benzimidazol-2-on-1-yl)-piperidin

NMR : 10,8 s (1H) ; 7,3-6,6 m (5H) ; 4,6-4,0 m (4H) ; 3,5-1,4 m + 3 s (19H)
IR : 1 688 $cm^{-1}$

Weiterhin werden analog der in Beispiel 1.9 angegebenen Vorschrift unter Verwendung entsprechender Mengen der betreffenden Ausgangsmaterialien die folgenden Verbindungen hergestellt :

## Beispiel 16

1-[(Benzo-1,4-dioxan-2-yl)-methyl]-4-(5-fluor-benzimidazol-2-on-1-yl)-piperidin

Freie Base : F. 174-178 °C
NMR : 11,0 s (1H) ; 7,3-6,5 m (7H) ; 4,5-3,6 m (4H) ; 3,3-1,5 m (10H)
IR : 1 688 $cm^{-1}$
Hydrochlorid, farbloses Pulver, F. > 169 °C (Zers.).

## Beispiel 17

1-[(Benzo-1,4-dioxan-2-yl)-methyl]-4-(5-methoxy-benzimidazol-2-on-1-yl)-piperidin

Freie Base : F. 177-181 °C
NMR : 11,0 s (1H) ; 7,3-6,2 m (7H) ; 4,5-3,6 m + s (7H) ; 3,3-1,5 m (10H)
IR : 1 680 $cm^{-1}$
Hydrochlorid, F. 220-223 °C (Zers.)

## Beispiel 18

1-[(5-Methyl-benzo-1,4-dioxan-2-yl)-methyl]-4-(5-fluor-benzimidazol-2-on-1-yl)-iperidin

NMR : 10,8 s (1H) ; 7,3-6,8 m (6H) ; 4,6-4,0 m (4H), 3,3-1,5 m + s (13H)
IR : 1 685 $cm^{-1}$

## Beispiel 19

1-[(5-Methyl-benzo-1,4-dioxan-2-yl)-methyl]-4-(5-methoxy-benzimidazol-2-on-1-yl)-piperidin

NMR : 10,9 s (1H) ; 7,3-6,4 m (6H) ; 4,6-4,0 m + s (7H) ; 3,3-1,5 m + s (13H)
IR : 1 690 $cm^{-1}$

## Beispiel 20

1-[(6-Methyl-benzo-1,4-dioxan-2-yl)-methyl]-4-(5-methyl-benzimidazol-2-on-1-yl)-piperidin

NMR : 10,8 s (1H) ; 7,3-6,4 m (6H) ; 4,6-4,0 m (4H) ; 3,3-1,5 m + s (16H)
IR : 1 685 $cm^{-1}$

## Beispiel 21

1-[(6-Methyl-benzo-1,4-dioxan-2-yl)-methyl]-4-(5-methoxy-benzimidazol-2-on-1-yl)-piperidin

NMR : 10,9 s (1H) ; 7,3-6,2 m (6H) ; 4,6-4,0 m + s (7H) ; 3,3-1,5 m + s (13H)
IR : 1 685 $cm^{-1}$

## Beispiel 22

1-[(6-Methyl-benzo-1,4-dioxan-2-yl)-methyl]-4-(5-fluor-benzimidazol-2-on-1-yl)-piperidin

Freie Base : F. 178-180 °C
NMR : 10,8 s (1H) ; 7,4-6,8 m (6H) ; 4,4-3,8 m (4H) ; 3,3-1,5 m + s (13H)
IR : 1 685 $cm^{-1}$
Hydrochlorid, F. > 170 °C (Zers.)

## Beispiel 23

1-[(6,7-Dimethyl-benzo-1,4-dioxan-2-yl)-methyl]-4-(5-fluor-benzimidazol-2-on-1-yl)-piperidin

NMR : 10,9 s (1H) ; 7,4-6,8 m (5H) ; 4,4-3,9 m (4H) ; 3,3-1,4 m + 2 s (16H)
IR : 1 685 $cm^{-1}$

Beispiel 24

1-[(6,7-Dimethyl-benzo-1,4-dioxan-2-yl)-methyl]-4-(5-methoxy-benzimidazol-2-on-1-yl)-piperidin

NMR : 10,9 s (1H) ; 6,9-6,0 m (5H) ; 4,6-3,9 m + s (7H) ; 3,3-1,4 m + 2 s (16H)
IR : 1 690 $cm^{-1}$

Beispiel 25

1-[(7-Chlor-benzo-1,4-dioxan-2-yl)-methyl]-4-(5-fluor-benzimidazol-2-on-1-yl)-piperidin

Freie Base : F. 152-154 °C
NMR : 10,9 s (1H) ; 7,3-6,6 m (6H) ; 4,6-4,0 m (4H) ; 3,5-1,5 m (10H)
IR : 1 685 $cm^{-1}$
Hydrochlorid, F. 233-236 °C (Zers.)

Beispiel 26

1-[(7-Chlor-benzo-1,4-dioxan-2-yl)-methyl]-4-(5-methoxy-benzimidazol-2-on-1-yl)-piperidin

NMR : 10,9 s (1H) ; 7,1-6,5 m (6H) ; 4,6-4,0 m + s (7H) ; 3,5-1,5 m (10H)
IR : 1 690 $cm^{-1}$

Beispiel 27

1-[(6,7-Dichlor-benzo-1,4-dioxan-2-yl)-methyl]-4-(5-methoxy-benzimidazol-2-on-1-yl)-piperidin

NMR : 11,0 s (1H) ; 7,3-6,3 m (5H) ; 4,6-4,0 m + s (7H) ; 3,5-1,5 m (10H)
IR : 1 690 $cm^{-1}$

Beispiel 28

1-[(6,7-Dichlor-benzo-1,4-dioxan-2-yl)-methyl]-4-(5-fluor-benzimidazol-2-on-1-yl)-piperidin

Freie Base : F. 199-201 °C
NMR : 11,0 s (1H) ; 7,3-6,9 m (5H) ; 4,6-4,0 m (4H) ; 3,5-1,5 m (10H)
IR : 1 688 $cm^{-1}$
Hydrochlorid, F. > 240 °C (Zers.)

Beispiel 29

1-[(6-Fluor-benzo-1,4-dioxan-2-yl)-methyl]-4-(5-chlor-benzimidazol-2-on-1-yl)-piperidin

Freie Base : F. 213-217 °C
NMR : 10,6 s (1H) ; 7,3-6,7 m (6H) ; 4,6-4,0 m (4H) ; 3,5-1,5 m (10H)
IR : 1 690 $cm^{-1}$
Hydrochlorid, F. 262-265 °C

Beispiel 30

1-[(7-Fluor-benzo-1,4-dioxan-2-yl)-methyl]-4-(5-chlor-benzimidazol-2-on-1-yl)-piperidin

Freie Base : F. 232-234 °C
NMR : 10,9 s (1H) ; 7,3-6,7 m (6H) ; 4,6-4,0 m (4H) ; 3,5-1,5 m (10H)
IR : 1 690 $cm^{-1}$
Hydrochlorid, F. 192-194 °C

Beispiel 31

1-[(6-Fluor-benzo-1,4-dioxan-2-yl)-methyl]-4-(5-fluor-benzimidazol-2-on-1-yl)-piperidin

Freie Base : F. 209 °C
NMR : 11,0 s (1H) ; 7,4-6,7 m (6H) ; 4,6-4,0 m (4H) ; 3,5-1,5 m (10H)
IR : 1 690 $cm^{-1}$
Hydrochlorid, F. 237 °C

Beispiel 32

1-[(7-Fluor-benzo-1,4-dioxan-2-yl)-methyl]-4-(5-fluor-benzimidazol-2-on-1-yl)-piperidin

Freie Base : F. 140 °C (Zers.)
NMR : 11,0 s (1H) ; 7,4-6,7 m (6H) ; 4,6-4,0 m (4H), 3,5-1,5 m (10H)
IR : 1 685 $cm^{-1}$
Hydrochlorid, F. 247-250 °C (Zers.)

Beispiel 33

1-[(6-Fluor-benzo-1,4-dioxan-2-yl)-methyl]-4-(5-methyl-benzimidazol-2-on-1-yl)-piperidin

NMR : 11,0 s (1H) ; 7,4-6,6 m (6H) ; 4,6-4,0 m (4H) ; 3,5-1,5 m + s (13H)
IR : 1 685 $cm^{-1}$

Beispiel 34

1-[(7-Fluor-benzo-1,4-dioxan-2-yl)-methyl]-4-(5-methyl-benzimidazol-2-on-1-yl)-piperidin

Freie Base : F. 231 °C
NMR : 11,0 s (1H) ; 7,4-6,6 m (6H) ; 4,6-4,0 m (4H) ; 3,5-1,5 m + s (13H)
IR : 1 685 $cm^{-1}$
Hydrochlorid, F. 292-295 °C

Beispiel 35

1-[(6-Fluor-benzo-1,4-dioxan-2-yl)-methyl]-4-(5-methoxy-benzimidazol-2-on-1-yl)-piperidin

NMR : 10,9 s (1H) ; 7,1-6,2 m (6H) ; 4,6-4,0 m + s (7H) ; 3,5-1,5 m (10H)
IR : 1 685 $cm^{-1}$

Beispiel 36

1-[(7-Fluor-benzo-1,4-dioxan-2-yl)-methyl]-4-(5-methoxy-benzimidazol-2-on-1-yl)-piperidin

NMR : 11,0 s (1H) ; 7,1-6,2 m (6H) ; 4,6-4,0 m + s (7H) ; 3,5-1,5 m (10H)
IR : 1 685 $cm^{-1}$

**Ansprüche** (für die Vertragsstaaten : BE, CH, DE, FR, GB, IT, LI, LU, NL, SE)

1. Verbindungen der Formel I

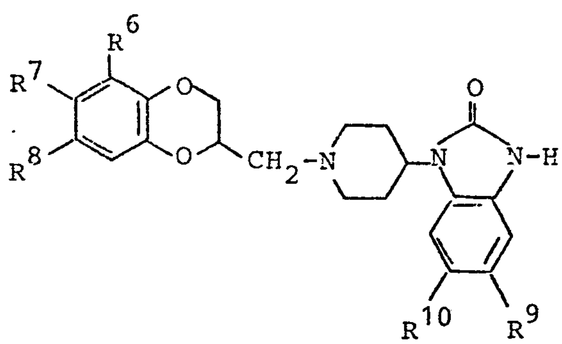

(I)

in der $R^6$ Wasserstoff oder Methyl, $R^7$ und $R^8$ unabhängig voneinander Wasserstoff, Fluor, Chlor oder Methyl, $R^9$ Fluor, Chlor, Methyl oder Methoxy und $R^{10}$ Wasserstoff oder Methyl bedeuten.

2. Verbindungen nach Anspruch 1, in denen $R^6$, $R^7$ und $R^{10}$ Wasserstoff, $R^8$ Wasserstoff oder Fluor und $R^9$ Fluor, Chlor, Methyl oder Methoxy bedeuten.

3. Verfahren zur Herstellung von Verbindungen der Formel I von Anspruch 1, dadurch gekennzeichnet, daß man

(1) eine Verbindung der Formel II

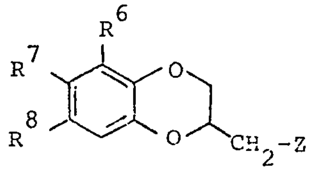

(II)

mit einer Verbindung der Formel III

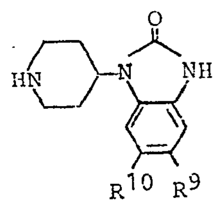

(III)

zu einer Verbindung der Formel I umsetzt, wobei alle Substituenten die in Anspruch 1 genannten Bedeutungen haben und Z für Halogen oder eine reaktionsfähige Estergruppe steht ; oder

(2) eine Verbindung der Formel IV

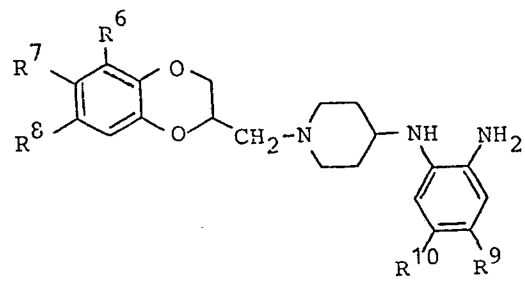

(IV)

mit einem reaktiven Kohlensäurederivat umsetzt, wobei alle Substituenten die in Anspruch 1 genannten Bedeutungen haben.

4. Arzneimittel, dadurch gekennzeichnet, daß es eine Verbindung der Formel I von Anspruch 1 in einer therapeutisch wirksamen Menge, gegebenenfalls zusammen mit üblichen pharmazeutischen Hilfs- und Trägerstoffen, enthält.

5. Arzneimittel nach Anspruch 4, dadurch gekennzeichnet, daß es eine neuroleptisch wirksame Menge einer Verbindung nach Anspruch 1 enthält.

6. Verwendung einer Verbindung der Formel I von Anspruch 1 zur Herstellung eines Arzneimittels.

7. Verwendung einer Verbindung der Formel I von Anspruch 1 zur Herstellung eines Neurolepticums.

**Anspruch** (für den Vertragsstaat AT)

Verfahren zur Herstellung von Verbindungen der Formel I

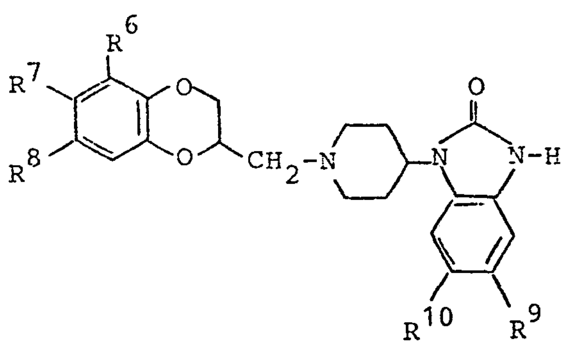

(I)

in der $R^6$ Wasserstoff oder Methyl, $R^7$ und $R^8$ unabhängig voneinander Wasserstoff, Fluor, Chlor oder Methyl, $R^9$ Fluor, Chlor, Methyl oder Methoxy und $R^{10}$ Wasserstoff oder Methyl bedeuten, und deren Salze mit physiologisch verträglichen Säuren, dadurch gekennzeichnet, daß man

(1) eine Verbindung der Formel II

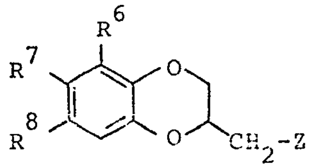

(II)

mit einer Verbindung der Formel III

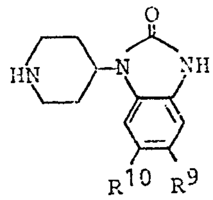

(III)

zu einer Verbindung der Formel I umsetzt, wobei alle Substituenten die vorstehend genannten Bedeutungen haben und Z für Halogen oder eine reaktionsfähige Estergruppe steht ; oder

(2) eine Verbindung der Formel IV

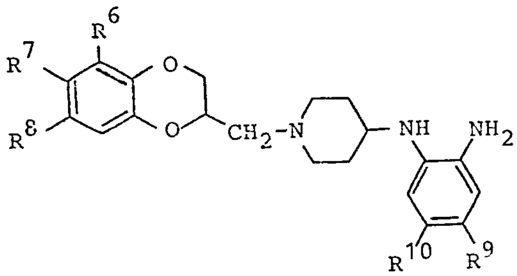

(IV)

mit einem reaktiven Kohlensäurederivat umsetzt, wobei alle Substituenten die vorstehend genannten Bedeutungen haben.

**Claims** (for the Contracting States : BE, CH, DE, FR, GB, IT, LI, LU, NL, SE)

1. Compounds of the formula I

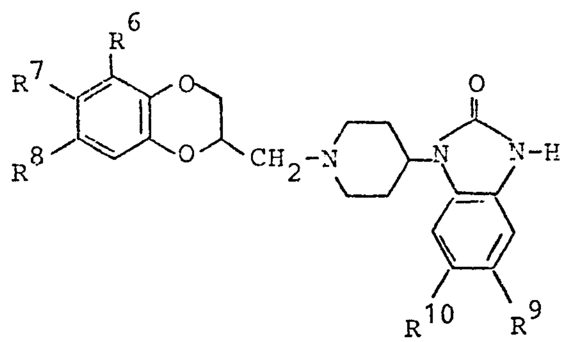

(I)

in which $R^6$ denotes hydrogen or methyl, $R^7$ and $R^8$ independently of one another denote hydrogen, fluorine, chlorine or methyl, $R^9$ denotes fluorine, chlorine, methyl or methoxy and $R^{10}$ denotes hydrogen or methyl.

2. Compounds as claimed in claim 1, in which $R^6$, $R^7$ and $R^{10}$ denote hydrogen, $R^8$ denotes hydrogen or fluorine and $R^9$ denotes fluorine, chlorine, methyl or methoxy.

3. A process for the preparation of compounds of the formula I as claimed in claim 1, which comprises

(1) reacting a compound of the formula II

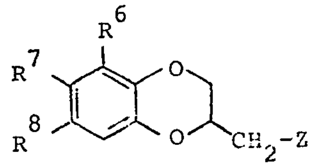

(II)

with a compound of the formula III

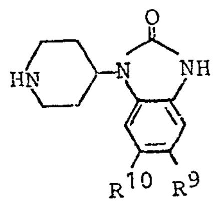

(III)

to give a compound of the formula I, all the substituents having the meaning given in claim 1 and 2 representing halogen or a reactive ester group ; or

(2) reacting a compound of the formula IV

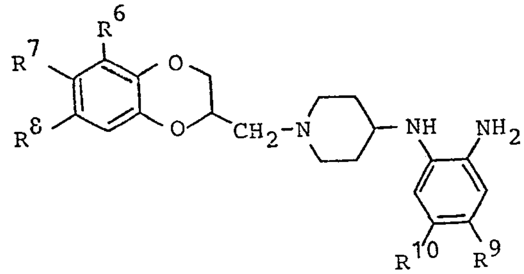

(IV)

with a reactive carbonic acid derivative, all the substituents having the meanings given in claim 1.

4. A medicament which contains a compound of the formula I as claimed in claim 1 in a therapeutically active amount, where appropriate together with conventional pharmaceutical auxiliaries and excipients.

5. A medicament as claimed in claim 4, which contains a neuroleptically active amount of a compound as claimed in claim 1.

6. The use of a compound of the formula I as claimed in claim 1 for the preparation of a medicament.

7. The use of a compound of the formula I as claimed in claim 1 for the preparation of a neuroleptically active medicament.

**Claim** (for the Contracting State AT)

A process for the preparation of compounds of the formula I

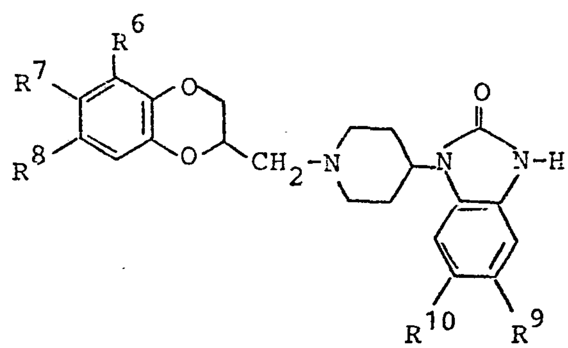

(I)

in which $R^6$ denotes hydrogen or methyl, $R^7$ and $R^8$ independently of one another denote hydrogen, fluorine, chlorine or methyl, $R^9$ denotes fluorine, chlorine, methyl or methoxy and $R^{10}$ denotes hydrogen or methyl and of their salts with physiologically acceptable acids, which comprises

(1) reacting a compound of the formula II

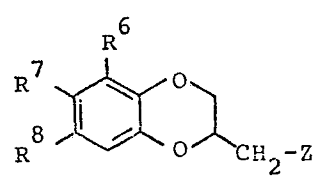

(II)

with a compound of the formula III

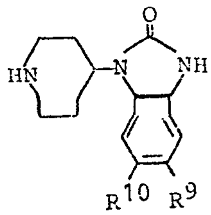

(III)

to give a compound of the formula I, all the substituents having the above mentioned meaning and Z representing halogen or a reactive ester group ; or

(2) reacting a compound of the formula IV

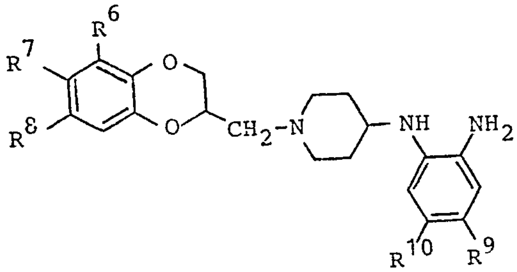

(IV)

with a reactive carbonic acid derivative, all the substituents having the meanings given above.

**Revendications** (pour les Etats contractants : BE, CH, DE, FR, GB, IT, LI, LU, NL, SE)

1. Composés de formule I

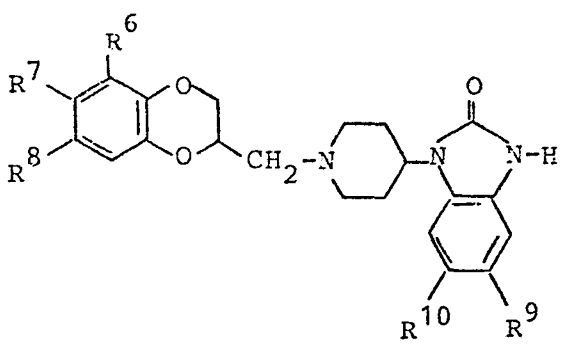

(I)

dans laquelle $R^6$ représente l'hydrogène ou un groupe méthyle, $R^7$ et $R^8$ indépendamment l'un de l'autre représentent l'hydrogène, le fluor, le chlore ou un groupe méthyle, $R^9$ représente le fluor ; le chlore, un groupe méthyle ou méthoxy et $R^{10}$ représente l'hydrogène ou un groupe méthyle.

2. Composés selon la revendication 1, dans lesquels $R^6$, $R^7$ et $R^{10}$ représentent l'hydrogène, $R^8$ représente l'hydrogène ou le fluor et $R^9$ représente le fluor, le chlore, un groupe méthyle ou méthoxy.

3. Procédé pour la préparation des composés de formule I selon la revendication 1, caractérisé en ce que

(1) on fait réagir un composé de formule II

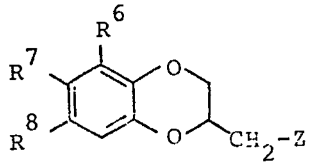

(II)

avec un composé de formule III

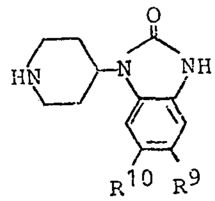

(III)

pour obtenir un composé de formule I, tous les substituants ayant les significations indiquées dans la revendication 1 et Z représentant un halogène ou un groupe ester réactif ; ou

(2) on fait réagir un composé de formule IV

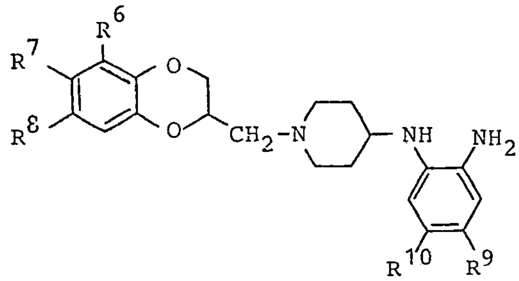

(IV)

avec un dérivé réactif de l'acide carbonique, tous les substituants ayant les significations indiquées dans la revendication 1.

4. Médicament, caractérisé en ce qu'il contient un composé de formule I selon la revendication 1 dans une quantité thérapeutiquement efficace, éventuellement avec des adjuvants et des véhicules pharmaceutiquement usuels.

5. Médicament selon la revendication 4, caractérisé en ce qu'il contient une quantité neuroleptique d'un composé selon la revendication 1.

6. Utilisation d'un composé de formule 1 selon la revendication 1 pour la préparation d'un médicament.

7. Utilisation d'un composé de formule I selon la revendication 1 pour la préparation d'un neuroleptique.

**Revendication** (pour l'Etat contractant AT)

Procédé pour la préparation de composés de formule I

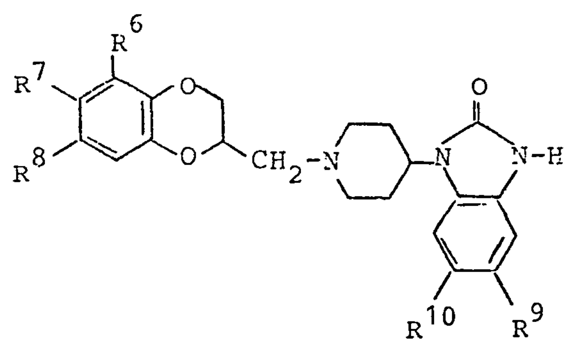

(I)

dans laquelle $R^6$ représente l'hydrogène ou un groupe méthyle, $R^7$ et $R^8$ représentent indépendamment l'un de l'autre, l'hydrogène, le fluor, le chlore ou un groupe méthyle, $R^9$ représente le fluor, le chlore, un groupe méthyle ou méthoxy et $R^{10}$ représente l'hydrogène ou un groupe méthyle, et de leurs sels avec des acides physiologiquement acceptables, caractérisé en ce que

(1) on fait réagir un composé de formule II

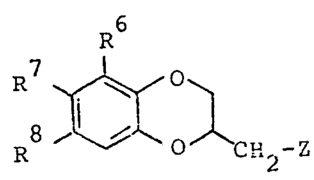

(II)

avec un composé de formule III

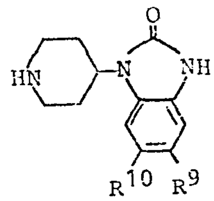

(III)

pour obtenir un composé de formule I, tous les substituants ayant les significations indiquées précédemment et Z représentant un halogène ou un groupe ester réactif ; ou

(2) on fait réagir un composé de formule IV

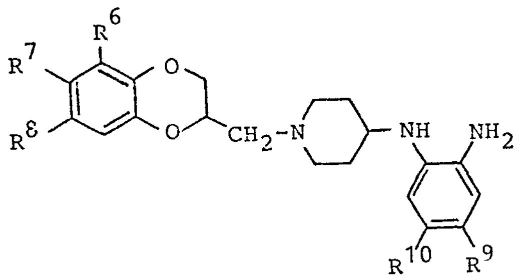

(IV)

avec un dérivé réactif de l'acide carbonique, tous les substituants ayant les significations précédemment indiquées.